# EUROPÄISCHE PATENTSCHRIFT

(11) **EP 3 764 952 B1**
(45) Veröffentlichungstag und Bekanntmachung des Hinweises auf die Patenterteilung: **03.05.2023**
(21) Anmeldenummer: 19710379.9
(22) Anmeldetag: 08.03.2019
(51) Int. Cl.: A61F 5/01

(54) **ORTHESE MIT SCHIENENARMBEFESTIGUNG MITTELS VERZAHNUNG**
ORTHOSIS COMPRISING BRACE ARM FIXATION USING GEARING
ORTHÈSE COMPRENANT UNE FIXATION DE BRAS DE RAIL AU MOYEN D'UNE DENTURE

(30) Priorität: 13.03.2018 DE 102018105780
(43) Veröffentlichungstag der Anmeldung: 20.01.2021
(73) Patentinhaber: Albrecht GmbH, 83071 Stephanskirchen (DE)
(72) Erfinder: OPAHLE, Hans-Georg, 83024 Rosenheim (DE)
(74) Vertreter: Flach Bauer & Partner Patentanwälte mbB
(86) Internationale Anmeldenummer: PCT/EP2019/055807
(87) Internationale Veröffentlichungsnummer: WO 2019/175039

(56) Entgegenhaltungen:
- EP-A1- 2 873 394
- WO-A1-2017/041993
- US-A1- 2011 314 637
- US-A1- 2014 308 065

## Beschreibung

Die Erfindung betrifft eine Orthese gemäß dem Oberbegriff des Anspruches 1.

Orthopädische Orthesen werden bekannterweise dafür verwendet, Gelenke wie beispielsweise Knie- oder Ellbogengelenke zu führen und zu stabilisieren und/oder Gliedma-βen mittels Federkraft in Extensions- oder Flexionsrichtung zu drängen.

Bei bekannten Orthesen dieser Art sind die Schwenkbereichgrenzen, innerhalb der die Schienenarme zwischen ihrer Extensions- und Flexionsendstellung relativ zueinander verschwenkt werden können, häufig mittels verstellbarer Anschlagstifte variabel einstellbar, die als Anschläge für den verschwenkbaren Schienenarm dienen. Üblicherweise werden diese Anschlagstifte in entsprechend angeordnete Bohrungen eingeschraubt.

Diese Art der Schwenkbereicheinstellung ist bei Orthesen, bei denen es auf eine sehr kleine Baugröße ankommt, beispielsweise bei Orthesen für Kleinkinder oder auch bei Orthesen für kleinere Tiere, nicht optimal, da die Bohrungen zum Einstecken oder Einschrauben der Anschlagstifte und die Anschlagstifte selbst einen gewissen Platz benötigen. Darüber hinaus ermöglicht diese bekannte Konstruktion in der Regel nur eine relativ grobe Winkelverstellung in Winkelschritten von 15° oder mehr.

Aus der US 2014/308065 A1 ist eine Orthese gemäß dem Oberbegriff des Anspruches 1 bekannt, bei welcher ein Gehäuse einer Krafteinheit mittels eines Schiebers drehfest mit einem Schienenarm gekoppelt und mit diesem verrastet werden kann.

Der Erfindung liegt von daher die Aufgabe zugrunde, eine Orthese der eingangs genannten Art mit einer Schwenkbereicheinstelleinrichtung zu schaffen, die einen möglichst geringen Platzbedarf hat und eine möglichst feine Verstellung des Schwenkbereichs ermöglicht.

Diese Aufgabe wird erfindungsgemäß durch eine Orthese mit den Merkmalen des Anspruches 1 gelöst. Vorteilhafte Ausführungsformen der Erfindung sind in den weiteren Ansprüchen beschrieben.

Bei der erfindungsgemäßen Orthese sind der erste Schienenarm und die Gehäuseabdeckung mittels einer Verzahnung drehfest koppelbar, die einen am ersten Schienenarm angeordneten ersten Zahnkranz und einen mit dem ersten Zahnkranz in lösbaren Eingriff bringbaren, mit der Gehäuseabdeckung gekoppelten zweiten Zahnkranz umfasst.

Mit Hilfe der Erfindung ist es möglich, auf Anschlagstifte und Bohrungen zum Einstecken der Anschlagstifte für die Schwenkbereichbegrenzung zu verzichten, da die Schwenkbereicheinstellung bzw. -begrenzung dadurch erfolgen kann, dass die beiden Zahnkränze in unterschiedlichen Drehwinkelstellungen des ersten Schienenarms (relativ zum Gehäuse) miteinander in drehfesten Eingriff gebracht werden können. Die Verstellung des Schwenkbereichs wird somit nicht wie bei bekannten Orthesen dadurch bewirkt, dass der Schwenkbereich des relativ zum Gehäuse schwenkbaren zweiten Schienenarms mittels unterschiedlich positionierbarer Anschlagstifte verändert wird, sondern dass die Position des ersten Schienenarms, der stationär an der Gehäuseabdeckung und damit am Gehäuse festlegbar ist, verändert wird. Der Schwenkbereich des zweiten Schienenarms relativ zum Gehäuse kann dabei immer gleich bleiben. Die Baugröße der erfindungsgemäßen Orthese kann dadurch bedeutend reduziert werden. Gewindebohrungen für das Einschrauben von Anschlagstiften entfallen, so dass auch keine Notwendigkeit besteht, diejenigen Schienenteile, in denen üblicherweise die Gewindebohrungen vorgesehen sind, mit einer bestimmten Dicke auszubilden, um einen ausreichenden Halt für die Schraubstifte zu gewährleisten. Ferner kann die Verzahnung ohne weiteres derart ausgebildet sein, dass die Schwenkbereichverstellung der Schienenarme in sehr kleinen Schritten, insbesondere auch von Schritten unter 15° erfolgen kann, da hierzu nur die Zähne der Verzahnung entsprechend fein, d.h. die Zahnteilung entsprechend groß, ausgebildet sein muss.

Gemäß einer vorteilhaften Ausführungsform besteht der am ersten Schienenarm angeordnete erste Zahnkranz aus einer um eine kreisförmige Öffnung des ersten Schienenarms umlaufenden Innenverzahnung, während der mit der Gehäuseabdeckung gekoppelte zweite Zahnkranz aus einer Außenverzahnung besteht, die auf einer mit der Gehäuseabdeckung fest verbundenen oder an dieser angeformten Zahnscheibe ausgebildet ist. Der erste Schienenarm ist hierbei in unterschiedlichen Drehwinkelpositionen auf die Zahnscheibe aufsteckbar. Eine derartige Ausbildung ermöglicht eine einfache und kostengünstige Herstellung der Verzahnung. Ferner ist die Schwenkbereicheinstellung auf sehr einfache und schnelle Weise dadurch möglich, dass der erste Schienenarm von der Zahnscheibe abgehoben und in der gewünschten neuen Winkelposition wieder auf die Zahnscheibe aufgesteckt wird.

Alternative Verzahnungen sind ebenfalls möglich, beispielsweise ein am ersten Schienenarm vorgesehener erster Zahnkranz, dessen Zähne sich axial in Richtung der Gehäuseabdeckung erstrecken und in entsprechend angeordnete Zahnvertiefungen eingreifen, die an der Gehäuseabdeckung vorgesehen sind.

Vorteilhafterweise weist der erste und zweite Zahnkranz Zähne mit einem Winkelabstand von 5-15°, vorzugsweise von 10° auf. Die Stellung des ersten Schienenarms relativ zum Gehäuse kann damit in Schritten von 5-15°, vorzugweise 10°, eingestellt werden.

Vorzugsweise besteht die Zahnscheibe aus einem Stirnzahnrad, das insbesondere eine zylinderförmige Außenkontur haben kann. Es ist es jedoch auch denkbar, Stirnzahnräder mit kegelförmiger Außenkontur zu verwenden, wobei dann die am ersten Schienenarm angeordnete Innenverzahnung entsprechend kegelförmig ausgebildet ist.

Gemäß einer vorteilhaften Ausführungsform weist das Gehäuse feststehende Schwenkbereichanschläge für den zweiten Schienenarm auf. Diese Schwenkbereichanschläge können beispielsweise aus entsprechenden Schultern oder Anschlagflächen bestehen, die in der Umfangswand des Gehäuses ausgebildet sind, was eine einfache, platzsparende und kostengünstige Konstruktion ermöglicht.

Vorteilhafterweise ist der erste Schienenarm axial an der Gehäuseabdeckung mittels eines zentralen Verschraubelements gehaltert, das in ein die Schwenkachse bildendes Achselements der Orthese einschraubbar ist. Hierdurch ist es nicht erforderlich, eine Schraubverbindung zwischen dem ersten Schienenarm und der Gehäuseabdeckung vorzusehen. Die Gehäuseabdeckung kann auf diese Weise aus einem relativ dünnen Plattenmaterial, insbesondere Blechmaterial, gefertigt werden.

Die Erfindung wird nachfolgend anhand der Zeichnungen beispielhaft näher erläutert. Es zeigen:
Figur 1: eine Seitenansicht der erfindungsgemäßen Orthese, wobei die Schienenarme verkürzt dargestellt sind;
Figur 2: eine Explosionsdarstellung der Orthese von Figur 1;
Figur 3: einen Schnitt durch die Orthese, wobei die Federkraftverstelleinrichtung und die Federkraftblockiereinrichtung nicht dargestellt sind;
Figur 4: einen Schnitt durch das Verschraubelement;
Figur 5: eine Draufsicht auf den ersten Schienenarm;
Figur 6: eine Draufsicht auf die Zahnscheibe;
Figur 7: eine räumliche Darstellung des Lagerelements;
Figur 8: eine Seitenansicht der Feder;
Figur 9: eine Draufsicht auf die Feder von Figur 8;
Figur 10: eine räumliche Darstellung des Achselements;
Figur 11: eine räumliche Darstellung der Federkraftverstelleinrichtung in Explosionsdarstellung;
Figur 12: eine räumliche Darstellung des Verstellzahnrads;
Figur 13: eine räumliche Darstellung des Federträgers;
Figur 14: einen Schnitt durch das Gehäuse längs der Linie XIV-XIV von Figur 15.
Figur 15: eine Ansicht von unten auf das Gehäuse;
Figur 16: einen Schnitt längs der Linie XVI-XVI von Figur 15.
Figur 17: eine Draufsicht auf die Steuerscheibe; und
Figur 18: einen Schnitt längs der Linie XVIII-XVIII von Figur 17.

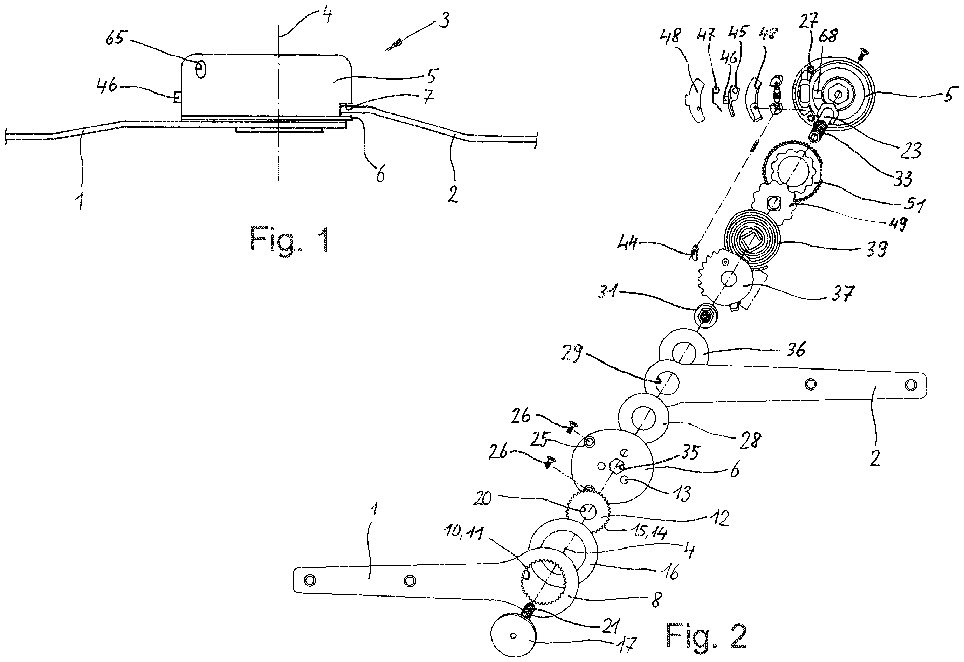

Figur 1 zeigt einen mittleren Gelenkabschnitt 3 einer erfindungsgemäßen Orthese mit einem ersten Schienenarm 1 und einem zweiten Schienenarm 2. Die beiden Schienenarme 1, 2 sind lediglich verkürzt dargestellt und weisen nicht dargestellte Befestigungseinrichtungen, insbesondere Klettbänder, Schalen oder dergleichen auf, mit denen die Schienenarme 1, 2 in bekannter Weise an mittels eines Körpergelenks verbundenen Gliedmaßen, beispielsweise am Ober- und Unterschenkel, Ober- und Unterarm etc., befestigt werden können.

Die Schienenarme 1, 2 sind im Gelenkabschnitt 3 gelenkig miteinander verbunden und um eine Schwenkachse 4 relativ zueinander schwenkbar. Ein schalenförmiges Gehäuse 5 ist auf seiner offenen, dem ersten Schienenarm 1 zugewandten Seite mittels einer plattenförmigen Gehäuseabdeckung 6 abgedeckt und über diese in einer nachfolgend noch näher beschriebenen Weise drehfest mit dem ersten Schienenarm verbunden.

Der zweite Schienenarm 2 erstreckt sich durch eine seitliche Ausnehmung 7 in der Umfangswand des Gehäuses 5 in das Innere des Gehäuses 5 hinein und ist dort in einer nachfolgend noch näher beschriebenen Weise schwenkbar gelagert. Die seitliche Ausnehmung 7 in der Gehäuseumfangswand erstreckt sich in Umfangsrichtung über einen derartigen Winkelbereich, dass sich der zweite Schienenarm 2 relativ zum Gehäuse 5 über einen gewünschten Winkelbereich, zweckmäßigerweise 90°-120°, schwenken lässt.

Im dargestellten Ausführungsbeispiel dient das Gehäuse 5 weiterhin zur Aufnahme eines nachfolgend noch näher erläuterten Federmechanismus sowie einer Federkraftverstelleinrichtung und einer Federkraftblockiereinrichtung. Es handelt sich beim dargestellten Ausführungsbeispiel somit um eine sog. Quengelschiene, die auf dem zweiten Schienenarm 2 eine Vorspannkraft in Flexions- oder Extensionsrichtung ausübt. Die vorliegende Erfindung ist jedoch nicht auf derartige Quengelschienen beschränkt, sondern kann auch bei Orthesen eingesetzt werden, die ohne Federkraft arbeiten.

Bei der erfindungsgemäßen Orthese wird der Schwenkbereich zwischen dem zweiten Schienenarm 2 und dem ersten Schienenarm 1 nicht dadurch variiert, dass der Schwenkbereich des zweiten Schienenarms 2 relativ zum Gehäuse 5 verändert wird, sondern dass der erste Schienenarm 1, der drehfest mit der Gehäuseabdeckung 6 und damit mit dem Gehäuse 5 verbindbar ist, in unterschiedlichen Drehwinkelstellungen an der Gehäuseabdeckung 6 festlegbar ist. Dies erfolgt mittels einer Verzahnung, die aus den Figuren 2, 5 und 6 ersichtlich ist und nachfolgend näher erläutert wird. Diese Verzahnung bildet somit eine Schwenkbereicheinstellvorrichtung.

Der erste Schienenarm 1 weist einen kreisscheibenförmigen Endbereich 8 mit einer mittigen kreisförmigen Öffnung 9 auf, die von einer Innenverzahnung 10 begrenzt ist. Diese Innenverzahnung 10 bildet einen ersten Zahnkranz 11.

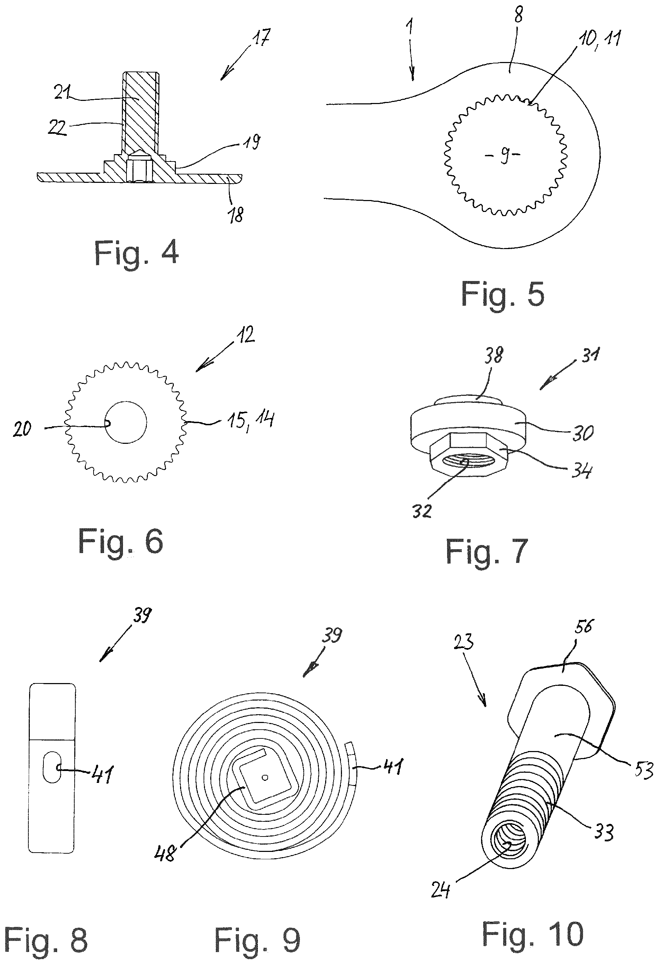

An derjenigen Seite der Gehäuseabdeckung 6, die dem ersten Schienenarm 1 zugewandt ist, ist eine Zahnscheibe 12 (Figuren 2 und 6) befestigt. Diese Befestigung kann beispielsweise dadurch erfolgen, dass die Zahnscheibe 12, insbesondere durch Bohrungen 13 der Gehäuseabdeckung 6 hindurch, an der Gehäuseabdeckung 6 festgeschweißt wird. Alternativ ist es auch möglich, die Zahnscheibe 12 einstückig mit der Gehäuseabdeckung 6 auszubilden.

Die kreisscheibenförmige Zahnscheibe 12 trägt an ihrem Außenumfang eine Außenverzahnung 14, die einen zweiten Zahnkranz 15 bildet. Die beiden Zahnkränze 11, 15 sind derart ausgebildet, dass der erste Schienenarm 1 auf die Zahnscheibe 12 aufgesteckt werden kann, wobei die beiden Zahnkränze 11, 15 miteinander kämmen und eine drehfeste Verbindung schaffen.

Es ist ersichtlich, dass die Winkelstellung des ersten Schienenarms 1 relativ zum Gehäuse 5 auf einfache Weise dadurch verändert werden kann, dass der erste Schienenarm 1 in unterschiedlichen Winkelpositionen auf die Zahnscheibe 12 aufgesteckt wird. Die Feinheit, d.h. der Winkelabstand, der Zähne der Innenverzahnung 10 und Au-βenverzahnung 14 bestimmt dabei die minimale Schrittweite der Winkelverstellung. Vorzugsweise ist die Verzahnung derart ausgebildet, dass der Winkelabstand zwischen benachbarten Zähnen 5-15°, insbesondere 10°, beträgt. Die Zahnscheibe 12 besteht im gezeigten Ausführungsbeispiel aus einem Stirnzahnrad.

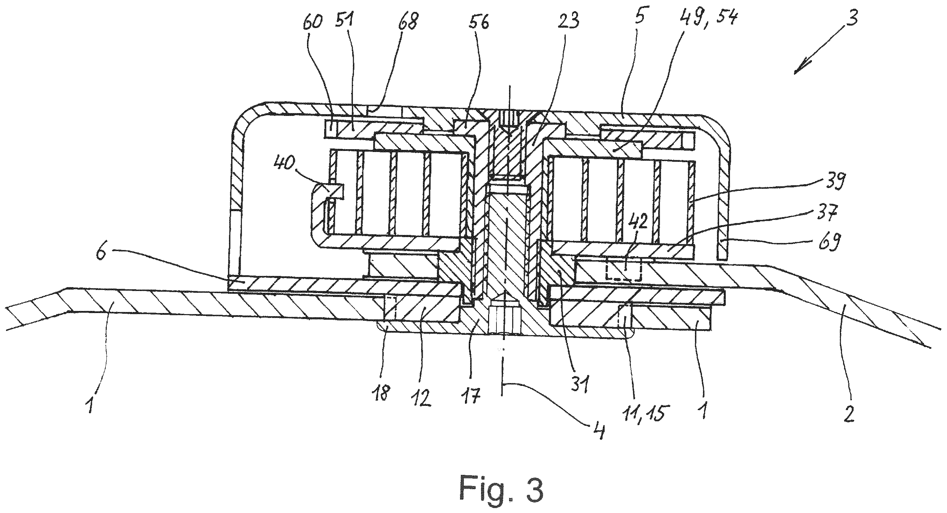

Anhand der Figuren 2 und 3 wird im Folgenden der Gesamtaufbau der Orthese näher beschrieben.

Zwischen dem Endbereich des ersten Schienenarms 1 und der Gehäuseabdeckung 6 ist eine Reibungsverminderungsscheibe 16 angeordnet, die beispielsweise aus Teflon bestehen kann. Der erste Schienenarm 1 wird an der Gehäuseabdeckung 6 mittels eines mit der Schwenkachse 4 fluchtenden Verschraubelements 17 gehalten, das in Figur 4 einzeln dargestellt ist. Das Verschraubelement 17 weist einen Kopf 18 mit einem Durchmesser auf, der größer als der Durchmesser der Öffnung 9 des ersten Schienenarms 1 ist, so dass der Kopf 18 den Endbereich 8 des ersten Schienenarms 1 im Randbereich der Öffnung 9 übergreift. Ein zylindrischer Absatz 19 des Verschraubelements 17 erstreckt sich in eine mittige Bohrung 20 der Zahnscheibe 12 hinein. Ein Gewindeschaft 21 ist mit einem Außengewinde 22 versehen, mit dem das Verschraubelement 17 in ein hülsenförmiges Achselement 23 (Figur 10) eingeschraubt werden kann, das hierzu ein entsprechendes Innengewinde 24 aufweist und fluchtend zur Schwenkachse 4 angeordnet ist.

Die Gehäuseabdeckung 6 ist platten- bzw. scheibenförmig ausgebildet. Im Randbereich der Gehäuseabdeckung 6 sind Löcher 25 zum Hindurchführen von Befestigungsschrauben 26 vorgesehen, die in Gewindelöcher 27 des Gehäuses 5 eingeschraubt werden können, um die Gehäuseabdeckung 6 fest mit dem Gehäuse 5 zu verbinden.

Auf der Innenseite der Gehäuseabdeckung 6 schließt sich, wie aus Figur 2 ersichtlich, eine Reibungsverminderungsscheibe 28, insbesondere Teflonscheibe, und der zweite Schienenarm 2 an.

Der zweite Schienenarm 2 weist in seinem überwiegend kreisringförmigen Endbereich eine Lagerbohrung 29 auf, mittels welcher der zweite Schienenarm 2 drehbar an einem zylinderförmigen Abschnitt 30 eines Lagerelementes 31 (Figur 7) gelagert ist.

Das Lagerelement 31 weist eine mittige, axial durchgehende Gewindebohrung 32 auf, mittels welcher das Lagerelement 31 auf einen Gewindeabschnitt 33 (Figur 10) des Achselements 23 aufgeschraubt werden kann. Auf der der Gehäuseabdeckung 6 zugewandten Seite des zylindrischen Abschnitts 30 weist das Lagerelement 31 einen mehrkantigen Fortsatz 34 mit verringertem Außendurchmesser auf, der in eine entsprechend geformte mehrkantige Öffnung 35 (Figur 2), die in der Gehäuseabdeckung 6 vorgesehen ist, eingeführt werden kann und dadurch ein Verdrehen des Lagerelementes 31 verhindert. Im gezeigten Ausführungsbeispiel ist der mehrkantige Fortsatz 34 sechskantförmig, es können jedoch auch beliebig andere Mehrkantformen oder andere Drehsicherungsmittel vorgesehen sein.

Wie aus Figur 2 ersichtlich, ist benachbart zum zweiten Schienenarm 2 eine weitere Reibungsverminderungsscheibe 36, insbesondere Teflonscheibe, angeordnet, die in gleicher Weise wie die Reibungsverminderungsscheibe 28 auf dem zylindrischen Abschnitt 30 des Lagerelementes 31 aufgesetzt ist (Figur 3).

Benachbart zur Reibungsverminderungsscheibe 36 ist eine Steuerscheibe 37 drehbar auf einem zylindrischen Fortsatz 38 des Lagerelementes 31 (Figur 7) gelagert. Die Steuerscheibe 37 dient einerseits dazu, die Federkraft einer zur Steuerscheibe 37 benachbarten Feder 39 auf den zweiten Schienenarm 2 zu übertragen, und andererseits zum Abkoppeln der Federkraft vom zweiten Schienenarm 2, wenn eine freie Schwenkbarkeit des zweiten Schienenarms 2 ohne Federkraftbeaufschlagung gewünscht wird.

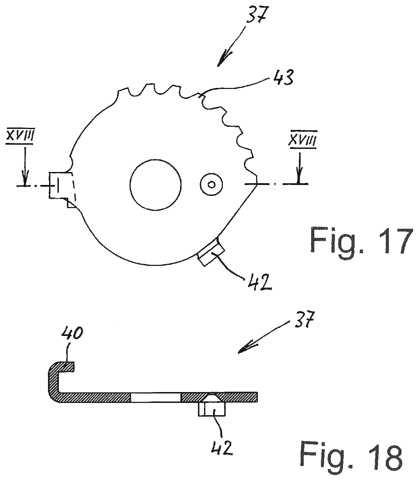

Die Steuerscheibe 37 weist, wie insbesondere aus den Figuren 3, 17 und 18 ersichtlich, eine Federeingriffslasche 40 auf, die in eine Öffnung 41 im äußeren Endbereich der Feder 39 eingreift. Über die Federeingriffslasche 40 wird somit die in Umfangsrichtung wirkende Kraft der Feder 39 auf die Steuerscheibe 37 übertragen, die auf dem Lagerelement 31 frei drehbar ist. Wie aus Figur 18 ersichtlich, steht die Federeingriffslasche 40 in Richtung der Feder 39, das heißt in Figur 18 nach oben, über die Hauptebene der Steuerscheibe 37 vor.

Die Steuerscheibe 37 weist ferner in ihrem Randbereich eine Mitnehmerlasche 42 auf, die mit dem zweiten Schienenarm 2 in Wirkverbindung ist. Die Mitnehmerlasche 42 steht über die Hauptebene der Steuerscheibe 37 in Richtung des zweiten Schienenarms 2 vor und übergreift eine Seitenfläche des Schienenarms 2. Die Mitnehmerlasche 42 drückt somit gegen die Seitenfläche des zweiten Schienenarms 2, wenn die Steuerscheibe 37 mittels der Feder 39 in Umfangsrichtung um die Schwenkachse 4 herum gedrängt wird. Je nach Richtung der Federkraft übt somit die Feder 39 auf den zweiten Schienenarm 2 eine Vorspannkraft in Extensions- oder Flexionsrichtung aus.

Die Steuerscheibe 37 weist ferner in ihrem Randbereich eine Verzahnung 43 auf, die zur Drehblockierung der Steuerscheibe 39 und damit zum Abkoppeln der Federkraft vom zweiten Schienenarm 2 dient. Mit dieser Verzahnung 43 kann eine Sperrklinke 44 (Figur 2) in und außer Sperreingriff gebracht werden. Die Betätigung der Sperrklinke 44 erfolgt über einen manuell in Umfangsrichtung verschiebbaren Sperrschieber 45, der in der Nähe der Umfangswand des Gehäuses 5 angeordnet ist und einen Betätigungsknopf 46 aufweist, der durch eine Öffnung in der Umfangswand hindurch nach außen vorragt und mit einem Finger betätigt werden kann. Am Sperrschieber 45 ist ein Federelement 47 gelagert, das von außen auf die Sperrklinke 44 drückt und die Sperrklinke 44 je nach Stellung des Sperrschiebers 45 in oder außer Eingriff mit der Verzahnung 43 hält. Weiterhin sind in Figur 2 noch zwei Füllstücke 48 dargestellt.

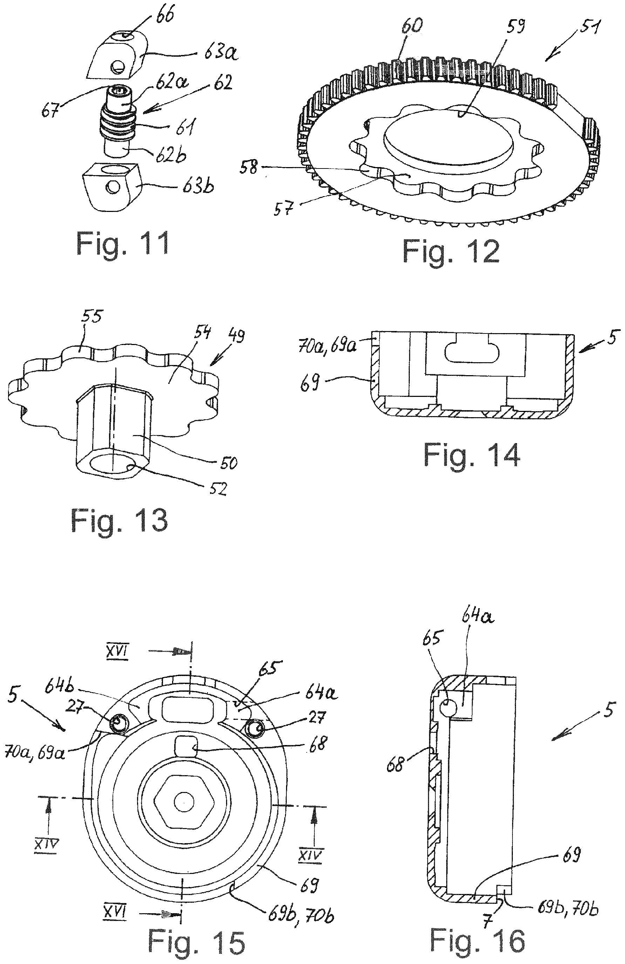

Bei der Feder 39 handelt es sich um eine Spiralfeder mit um die Schwenkachse 4 herumlaufenden Federwindungen. Der innere Endbereich 48 der Feder 39 (Figur 9) ist vierkantförmig geformt und drehfest mit einem Federträger 49 (Figur 13) verbunden.

Wie aus Figur 13 ersichtlich, weist der Federträger 49 hierzu einen Federeingriffabsatz 50 mit einer Außenkontur auf, die an die Innenkontur des inneren Endbereichs 48 der Feder 39 angepasst ist. Der Endbereich 48 der Feder 39 kann dadurch auf den Federeingriffabsatz 50 drehfest aufgesteckt werden.

Der Federträger 49 dient dazu, den inneren Endbereich 48 der Feder 39 stationär relativ zum Gehäuse 5 und damit zum ersten Schienenarm 1 zu halten. Weiterhin dient der Federträger 49 zusammen mit einem drehfest mit dem Federträger 49 koppelbaren Verstellzahnrad 51 zum Einstellen der Vorspannkraft der Feder 39. Hierzu weist der Federträger 49 eine axiale Lagerbohrung 52 auf, mittels welcher der Federträger 49 auf einem zylindrischen Lagerabschnitt 53 des Achselements 23 (Figur 10) drehbar gelagert ist.

Mit dem Federeingriffabsatz 50 des Federträgers 49 ist eine Kupplungsscheibe 54 fest verbunden. Die Kupplungsscheibe 54 weist eine wellen- oder zahnförmige Außenkontur 55 auf, die zur drehfesten, jedoch axial lösbaren Kopplung mit dem Verstellzahnrad 51 dient.

In Axialrichtung wird der Federträger 49 durch einen Kopf 56 des Achselements 23 (Figur 10) gehalten, der die Kupplungsscheibe 54 des Federträgers 49 übergreift.

Figur 12 zeigt das Verstellzahnrad 51 schräg von unten, das heißt von derjenigen Seite her, die mit dem Federträger 49 formschlüssig in drehfesten Eingriff bringbar ist. Hierzu weist das Verstellzahnrad 51 eine von seiner Unterseite ausgehende, kreisförmige Vertiefung 57 mit einem wellenförmigen Umfangsrand 58 auf, der an die Außenkontur 55 des Federträgers 49 angepasst ist, so dass das Verstellzahnrad 51 drehfest auf die Kupplungsscheibe 54 des Federträgers 49 aufsteckbar ist. Eine mittige Durchgangsöffnung 59 hat einen größeren Durchmesser als der Kopf 56 des Achselements 23, so dass der in die Durchgangsöffnung 59 hineinragende Kopf 56 das Drehen des Verstellzahnrads 51 um die Schwenkachse 4 herum nicht behindert.

Das Verstellzahnrad 51 weist weiterhin eine Stirnverzahnung 60 auf, die mit einem Schneckengewinde 61 einer

Schnecke 62 (Figur 11) in Eingriff ist. Die Schnecke 62 ist mit zylindrischen Endabschnitten 62a, 62b in Lagerelementen 63a, 63b drehbar gelagert. Die Lagerelemente 63a, 63b sind in seitliche, taschenartige Vertiefungen 64a, 64b des Gehäuses 5 (Figuren 15 und 16) derart eingesetzt, dass die Längs- bzw. Drehachse der Schnecke 62 quer zur Schwenkachse 4 verläuft.

Das Schneckengewinde 61 ist selbsthemmend. Die durch die Schnecke 62 eingestellte Drehposition des Verstellzahnrads 51 und damit des Federträgers 49 relativ zum Gehäuse 5 ist damit festgelegt und kann sich damit ohne manuellen Eingriff nicht ändern. Andererseits kann die Vorspannkraft der Feder 39 erhöht oder verringert werden, indem die Drehposition des Verstellzahnrads 51 und damit des Federträgers 49 und des inneren Endbereichs 48 der Feder 39 durch manuelles Drehen der Schnecke 62 verändert wird. Im gezeigten Ausführungsbeispiel kann die Schnecke 62 mittels eines nicht dargestellten Inbusschlüssels gedreht werden, der durch ein seitliches Loch 65 des Gehäuses 5 (Figuren 15 und 16) sowie durch ein Loch 66 des Lagerelementes 63a hindurch (Figur 11) in eine axiale Inbusvertiefung 67 der Schnecke 62 eingeführt werden kann.

Auf der Oberseite des Verstellzahnrads 51 kann eine auf einem Kreisbogen um die Drehachse herum angeordnete Zahlenskala vorgesehen sein, die durch ein Schauloch 68 des Gehäuses 5 (Figuren 15 und 16) hindurch sichtbar ist und dem Benutzer die eingestellte Vorspannung anzeigt.

Wie aus den Figuren 14 bis 16 ersichtlich, weist das Gehäuse 5 eine Umfangswand 69 mit Anschlagflächen 69a, 69b auf, die als Schwenkbereichanschläge 70a, 70b ausgebildet sind und zur Begrenzung des Schwenkbereichs des zweiten Schienenarms 2 dienen. Im Bereich zwischen den Anschlagflächen 69a, 69b ist der zweite Schienenarm 2 verschwenkbar. Der zum Verschwenken des zweiten Schienenarms 2 erforderliche Freiraum zwischen der Umfangswand 69 und der Gehäuseabdeckung 6 wird dadurch erreicht, dass die Umfangswand 69 im Bereich zwischen den Anschlagflächen 69a, 69b einen entsprechenden Abstand zur Gehäuseabdeckung 6 hat, wie auch aus Figur 3 ersichtlich.

## Patentansprüche

1. Orthese, umfassend
- einen ersten und zweiten Schienenarm (1, 2), die in einem Gelenkabschnitt (3) gelenkig miteinander verbunden und um eine Schwenkachse (4) schwenkbar sind,
- ein im Gelenkabschnitt (3) angeordnetes Gehäuse (5), das mit dem ersten Schienenarm (1) stationär gekoppelt ist, während der zweite Schienenarm (2) relativ zum Gehäuse (5) schwenkbar ist,
- eine am Gehäuse (5) befestigte Gehäuseabdeckung (6), mit der eine offene Seite des Gehäuses (5) zumindest überwiegend verschließbar ist,
- eine Schwenkbereicheinstelleinrichtung zum Einstellen des Schwenkbereichs der ersten und zweiten Schienenarme (1, 2) relativ zueinander,
**dadurch gekennzeichnet, dass** der erste Schienenarm (1) und die Gehäuseabdeckung (6) mittels einer Verzahnung drehfest koppelbar sind, die einen am ersten Schienenarm (1) angeordneten ersten Zahnkranz (11) und einen mit dem ersten Zahnkranz (11) in lösbaren Eingriff bringbaren, mit der Gehäuseabdeckung (6) gekoppelten zweiten Zahnkranz (15) umfasst.

2. Orthese nach Anspruch 1, **dadurch gekennzeichnet, dass** der am ersten Schienenarm (1) angeordnete erste Zahnkranz (11) aus einer um eine kreisförmige Öffnung (9) des ersten Schienenarms (1) umlaufenden Innenverzahnung (10) besteht, und dass der mit der Gehäuseabdeckung (6) gekoppelte zweite Zahnkranz (15) aus einer Außenverzahnung (14) besteht, die auf einer mit der Gehäuseabdeckung (6) fest verbundenen oder an dieser angeformten Zahnscheibe (12) ausgebildet ist, wobei der erste Schienenarm (1) in unterschiedlichen Drehwinkelpositionen auf die Zahnscheibe (12) aufsteckbar ist.

3. Orthese nach Anspruch 1 oder 2, **dadurch gekennzeichnet, dass** der erste und zweite Zahnkranz (11, 15) Zähne mit einem Winkelabstand von 5° bis 15° aufweist.

4. Orthese nach Anspruch 3, **dadurch gekennzeichnet, dass** der Winkelabstand der Zähne 10° beträgt.

5. Orthese nach einem der vorhergehenden Ansprüche, **dadurch gekennzeichnet, dass** die Zahnscheibe (12) aus einem Stirnzahnrad besteht.

6. Orthese nach einem der vorhergehenden Ansprüche, **dadurch gekennzeichnet, dass** das Gehäuse (5) feststehende Schwenkbereichanschläge (70a, 70b) für den zweiten Schienenarm (2) aufweist.

7. Orthese nach Anspruch 6, **dadurch gekennzeichnet, dass** die Schwenkbereichanschläge (70a, 70b) aus Anschlagflächen (69a, 69b) bestehen, die in der Umfangswand (69) des Gehäuses (5) ausgebildet sind.

8. Orthese nach einem der vorhergehenden Ansprüche, **dadurch gekennzeichnet, dass** der erste Schienenarm (1) axial an der Gehäuseabdeckung (6) mittels eines zentralen Verschraubelementes (17) gehaltert ist, das in ein die Schwenkachse (4) bildendes Achselement (23) der Orthese einschraubbar ist.

## Claims

1. Orthosis, comprising:
- a first and second brace arm (1, 2) which are articulatedly connected to one another in an articulated section (3) and can be swivelled about a swivel axis (4),
- a housing (5), arranged in the articulated section (3), which is fixedly coupled to the first brace arm (1), while the second brace arm (2) can be swivelled relative to the housing (5),
- a housing cover (6) attached to the housing (5), with which an open side of the housing (5) can be at least predominantly closed,
- a swivel range adjusting device for adjusting the swivel range of the first and second brace arms (1, 2) relative to each other,
**characterised in that** the first brace arm (1) and the housing cover (6) can be coupled in a rotationally fixed manner by means of a gearing which comprises a first ring gear (11), located on the first brace arm (1), and a second ring gear (15) that can be brought into releasable engagement with the first ring gear (11) and that is coupled to the housing cover (6).

2. Orthosis according to claim 1, **characterised in that** the first ring gear (11) arranged on the first brace arm (1) consists of an internal gear (10) which surrounds a circular opening (9) in the first brace arm (1), and **in that** the second ring gear (15) coupled to the housing cover (6) consists of an external gear (14) which is formed on a toothed washer (12) fixedly connected to the housing cover (6) or integrally moulded thereon, wherein the first brace arm (1) can be fitted onto the toothed washer (12) in different positions of angular rotation.

3. Orthosis according to claim 1 or 2, **characterised in that** the first and second ring gears (11, 15) have teeth with an angular spacing of from 5° to 15°.

4. Orthosis according to claim 3, **characterised in that** the angular spacing of the teeth is 10°.

5. Orthosis according to one of the preceding claims, **characterised in that** the toothed washer (12) consists of a spur gear.

6. Orthosis according to one of the preceding claims, **characterised in that** the housing (5) has fixed swivel range stops (70a, 70b) for the second brace arm (2).

7. Orthosis according to claim 6, **characterised in that** the swivel range stops (70a, 70b) consist of abutment surfaces (69a, 69b) which are formed in the peripheral wall (69) of the housing (5).

8. Orthosis according to one of the preceding claims, **characterised in that** the first brace arm (1) is held axially on the housing cover (6) by means of a central screw element (17) which can be screwed into an axle element (23) of the orthosis forming the swivel axis (4).

## Revendications

1. Orthèse comprenant
- un premier et un deuxième bras de rail (1, 2) qui sont reliés ensemble de manière articulée dans un segment d'articulation (3) et sont pivotables autour d'un axe de pivotement (4),
- un boitier (5) disposé dans le segment d'articulation (3) qui est couplé de manière fixe avec le premier bras de rail (1), tandis que le deuxième bras de rail (2) est pivotable par rapport au boitier (5),
- un couvercle de boitier (6) fixé sur le boitier (5) avec un côté ouvert du boitier (5) pouvant être fermé au moins la plupart du temps,
- un dispositif de réglage de zones de pivotement permettant de régler la zone de pivotement des premier et deuxième bras de rail (1, 2) l'un par rapport à l'autre,
**caractérisée en ce que** le premier bras de rail (1) et le couvercle de boitier (6) peuvent être couplés en ne pouvant pas tourner au moyen d'un système d'engrenage qui comprend une première couronne dentée (11) disposée sur le premier bras de rail (1) et une deuxième couronne dentée (15) couplée avec le couvercle de boitier (6) pouvant être mise en prise de manière amovible avec la première couronne dentée (11).

2. Orthèse selon la revendication 1, **caractérisée en ce que** la couronne dentée (11) disposée sur le premier bras de rail (1) est constituée d'une indentation intérieure (10) parcourant un orifice (9) en forme de cercle du premier bras de rail (1) et que la deuxième couronne dentée (15) couplée avec le couvercle de boitier (6) est constituée d'une indentation extérieure (14) qui est reliée solidement avec le couvercle de boitier (6) ou est formée sur ce disque denté (12) surmoulé, où le premier bras de rail (1) peut être posé sur le disque denté (12) dans diverses positions de rotation angulaire.

3. Orthèse selon la revendication 1 ou la revendication 2, **caractérisée en ce que** les première et deuxième couronnes dentées (11, 15) présentent des dents avec un écart angulaire de 5 ° à 15 °.

4. Orthèse selon la revendication 3, **caractérisée en ce que** l'écart angulaire des dents est de 10 °.

5. Orthèse selon l'une des revendications précédentes, **caractérisée en ce que** le disque denté (12) est constitué d'un engrenage droit.

6. Orthèse selon l'une des revendications précédentes, **caractérisée en ce que** le boitier (5) présente des butées de zones de pivotement (700a, 70b) fixes pour le deuxième bras de rail (2).

7. Orthèse selon la revendication 6, **caractérisée en ce que** les butées de zones de pivotement (70a, 70b) sont constituées de surfaces de butées (69a, 69b) qui sont formées dans la paroi périphérique (69) du boitier (5).

8. Orthèse selon l'une des revendications précédentes, **caractérisée en ce que** le premier bras de rail (1) est tenu axialement sur le couvercle de boitier (6) au moyen d'un élément de vissage (17) central qui peut être vissé dans un élément d'axe (23) de l'orthèse formant l'axe de pivotement (4).
